# EUROPEAN PATENT APPLICATION

(11) **EP 1 652 519 A1**
(43) Date of publication of application: **03.05.2006**
(21) Application number: 05023453.3
(22) Date of filing: 27.10.2005
(51) Int. Cl.: A61K 31/198, A61K 33/04, A23L 1/304, A61P 39/06, A61P 35/00

(54) **Seleniferous composition for producing antioxidase in vivo**

(30) Priority: 28.10.2004 JP 2004314376
(71) Applicant: Nasu, Tamie, Matsumoto-shi, Nagano 390-0802 (JP); Takeda, Iwao, Kamiina-gun, Nagano 399-4601 (JP); Yamanoshita, Osamu, Showa-ku Nagoya-shi Aichi 466-8550 (JP)
(72) Inventor: Nasu, Tamie, Matsumoto-shi, Nagano 390-0802 (JP); Takeda, Iwao, Kamiina-gun, Nagano 399-4601 (JP); Yamanoshita, Osamu, Showa-ku Nagoya-shi Aichi 466-8550 (JP)
(74) Representative: Bauer, Friedrich

(57) **Abstract**

Seleniferous composition is provided which comprises Se-methylseleno-cysteine (MeSeCys: SeCH₃-CH₂-CHNH₂-COOH) or derivative thereof. When human or animal body ingests the seleniferous composition, their living body naturally produces enzymes such as superoxide dismutase (SOD), glutathione peroxidase (GPx) and glutathione-S-transferase (GST) for decomposing active oxygen to positively prevent and treat cancer.

## Description

### TECHNICAL FIELD

The present invention relates to a selenic composition, and particularly, a seleniferous composition capable of serving to generate an enzyme which produces antioxidative activity in a living body.

### BACKGROUND ART

It has been reported that production of active oxygen in vivo may have a harmful effect on causes of human's diseases such as cancer, myocardial infarction, cerebrovascular disorder and dementia. In accordance with the energy metabolism in a living body, ingesta such as carbohydrates, lipids or protein is biologically oxidized into mainly carbon dioxide and water, producing physiological heat and injurious active oxygen. Some kind of active oxygen is an unstable and highly reactive free radical. On the other hand, active oxygen is inhaled together with air including exhaust from engines and cigarette smoke so that active oxygen produces the strong oxidation which may denature DNA (deoxyribonucleic acid) and genetic information contained therein to cause cellular mutation and canceration. In order to treat and prevent malignant disease such as cancer resulted from active oxygen, many attempts have been made to propose various antioxidative material.

Selenium, the one of essential trace elements for living bodies, has the narrow transitional range between required and toxic levels, and insufficient or excessive amount of ingested selenium would cause considerable damage on a human body. Over some areas where plants are cultivated in soil of poor selenium and people ingest such plants, high mortality caused by cancer because of lack of selenium is reported, and skeletal muscle disorder, cardiomyopathy, coronary artery disease have broken out. Selenium is generally available as an anticancer agent and a supplement, and by way of example of seleniferous anticancer agent, Japanese Patent Disclosure No. 9-227378 demonstrates a pharmaceutical composition comprising a seleniferous cyanine dye to remedy cancer or tumor.

One of biological mechanisms for preventing generation, multiplication and transformation of cancer cells, utilizes enzymes such as superoxide dismutase (SOD), glutathione peroxidase (GPx) and glutathione-S-transferase (GST) which may decompose and detoxify active oxygen. Active oxygen like a superoxide (O₂⁻) considered as cause of cancer (malignant tumor) is decomposed or counteracted into a hydrogen peroxide (H₂O₂) in cytoplasm by superoxide dismutase (SOD), and then into water (H₂O) by glutathione peroxidase (GPx). Also, glutathione-S-transferase (GST) catalyzes a conjugation between active oxygen and glutathione (cysteine peptide) to detoxicate carcinogenic substances.

Japanese Patent Disclosure No. 5-30966 represents a SOD or superoxide dismutase which has the covalent bond between metal chelate and superoxide dismutase effective to prevent generation of unnecessary oxygen radicals in blood and inhibit aging and carcinogenesis.

The seleniferous anticancer agent shown by Japanese Patent Disclosure No. 9-227378 is prepared from a cyanine dye, however, disadvantageously gives rise to a serious problem against safety. Specifically, since selenium has a high toxicity and indicates only an extremely narrow harmless content range, toxicity of selenium can hardly be thoroughly inhibited in the form of cyanine compound, and in fact, the seleniferous anticancer agent may tend to produce the side effect induced by selenic substance. Also, the compound shown in Japanese Patent Disclosure No. 5-30966 comprises superoxide dismutase (SOD) capable of resolving active oxygen for direct administration of the compound into a living body by an injection, however, superoxide dismutase is disadvantageous because it is susceptible to deterioration by other substances on the pathway to carcinomatous site. Specifically, protein superoxide dismutase may be decomposed in vivo by proteolytic enzymes so that, in some case, it may be degraded with the impaired resolvability of active oxygen before reaching carcinomatous sites, and therefore, the enzymes cannot effectively produce any effective antioxidative and anticancer actions.

Accordingly, an object of the present invention is to provide a seleniferous composition for producing enzymes capable of producing a superior antioxidative or anticancer action. Another object of the present invention is to provide a seleniferous composition harmless and nonpoisonous to a human body. A still further object of the present invention is to provide a seleniferous composition capable of directly synthesizing in a living body an enzyme for metabolizing or decomposing carcinogens into harmless substances.

### DISCLOSURE OF INVENTION

The seleniferous composition according to the present invention comprises a Se-methylselenocysteine or derivative thereof. When a human or animal body ingests the seleniferous composition inclusive of Se-methylselenocysteine (MeSeCys: SeCH₃-CH₂-CHNH₂-COOH) or derivative thereof, their kidney produces a large amount of superoxide dismutase (SOD) as enzymes for disintegrating active oxygen. The antioxidative effect of superoxide dismutase (SOD) detoxifies active oxygen as a cause of cancer to effectively prevent and treat the generation and transformation of kidney cancer, renal pelvic tumor or the like, while improving antibody production, immune strength and resistance of human body against active oxygen for the enhancement of kidney function. In addition, unlike metallic selenium (Se), selenite ion (SeO₃²⁻) or selenate ion (SeO₄²⁻), Se-methylselenocysteine or derivative thereof can reduce or detoxyfy the toxicity of selenic composition to human body, and it is possible for a living body to increase the intake of detoxified seleniferous composition providing a high carcinostatic action. Furthermore, unlike administration of enzymes for degrading carcinogen via oral route or into blood, the seleniferous composition according to the present invention can directly synthesize in vivo superoxide dismutase (SOD) for detoxifying carcinogen without the need of considering on which pathway should be selected to lead enzymes to carcinomatous sites and on what should be done to prevent the degrading effect on enzymes by substances on the pathway to lesions.

### BRIEF DESCRIPTION OF THE DRAWINGS

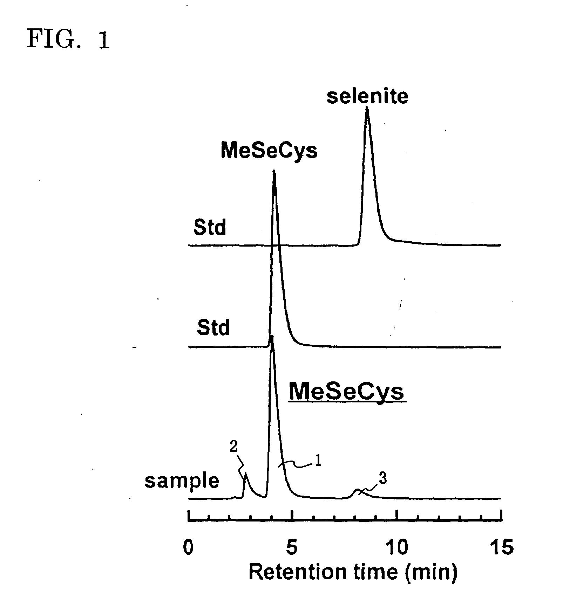

Figure 1 shows a graph of mass spectrography of enzymes analyzed by high performance liquid chromatograph and inductively coupled plasma mass spectrometry.

### DETAILED DESCRIPTION OF THE INVENTION

Detailed embodiments of the seleniferous composition according to the present invention are described hereinafter. The seleniferous composition comprises Se-methylselenocysteine shown in the following chemical formula (1):

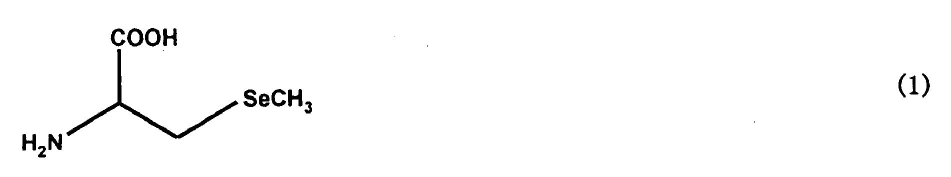

Derivative of Se-methylselenocysteine has a metallic or nonmetallic element, or organic group such as alkyl and aryl groups substituted for hydrogen (H) of carboxyl group (-COOH) shown in structural formula (1) of Se-methylselenocysteine.

The seleniferous composition comprises at least an amino compound which may comprise one or more radicals of amino acid, amino acid residues (-(HN-R-CO)ₙ-) or amino acid groups (H₂N-R-CO- or H₂N-R-COO-) selected from the group consisting essentially of glycine, alanine, valine, arginine, lysine, aspartic acid, glutaminic acid, serine, threonine, tyrosine, methionine and cystine. In particular, the amino compound preferably includes glutamyl group of glutamic acid. In some cases, Se-methylselenocysteine alone cannot produce enzymes for degrading various types of active oxygens generated in vivo or ingested from the outside. In an embodiment of the present invention, the amino compound is added to Se-methylselenocysteine to prepare the strong enzymes for converting, attenuating or detoxifying active oxygen into innocence. With the amino compound involved, the composition can produce the enzymes capable of converting various kind or type of active oxygen into innocent or inactive ones. Such amino compounds used in the present invention may be extracted from silk scouring solution.

The glutamyl compound preferably includes glutamylmethylseleno-cysteine, and particularly, γ-glutamylmethylselenocysteine shown in the following chemical formula (2):

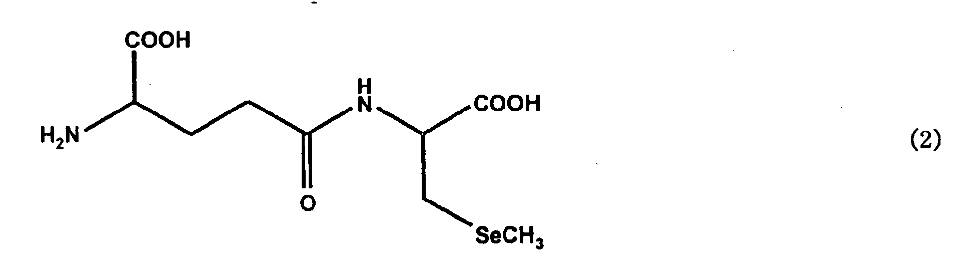

Glutamylmethylselenocysteine can be prepared by chemically bonding amino group (-NH₂) of methylselenocysteine and carboxyl group (-COOH) of glutaminic acid (COOH-CH₂-CH₂-CNH₂COOH) under dehydration to have the glutamyl group (-CO-CH₂-CH₂-CNH₂COOH) which promotes to generate a large amount of useful enzymes such as superoxide dismutase (SOD) and simultaneously positively attenuates or neutralizes toxicity of selenium for improvement in safety of taking seleniferous composition. The seleniferous composition according to the present invention comprises, on the weight basis, 40 to 120 parts of Se-methylselenocysteine or derivative thereof; and 4 to 20 parts of a glutamylmethylselenocysteine. Amount of Se-methylselenocysteine or derivative thereof under 40 parts will degrade the ability for decomposing active oxygen by the composition, and the amount thereof above 120 parts will adversely increase the toxicity of the compounds. In addition, the amount of glutamylmethylseleno-cysteine under 4 parts will heighten toxicity of the compounds, and the amount of glutamylmethylselenocysteine over 20 parts will control the toxicity of the compounds, but deteriorate the resolving ability of active oxygen. To further raise the resolving ability of active oxygen, the seleniferous composition according to the present invention may comprise, on the weight basis, 2 to 15 parts of selenious acid shown in the following chemical formula (3):

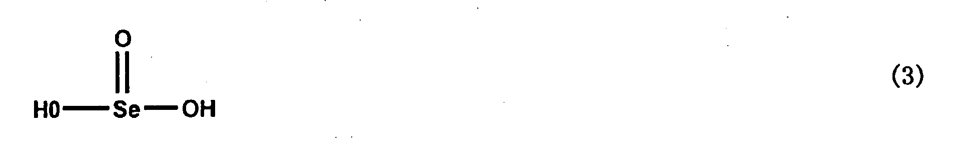

To prepare the seleniferous composition according to the present invention, plants or agricultural products such as daikon sprouts are cultivated with a seleniferous chelate fertilizer to grow them while absorbing the seleniferous chelate, and then harvested and dried. Seleniferous chelate fertilizer may include saccharic acid, such as pentaric, hexalic and gluconic acids. After milling the products, the resultant powder is formed into a medicine or chemicals of tablet, capsule, powder or granule for dissolution and absorption of the composition in stomach and intestines to finish the seleniferous composition according to the present invention. However, the composition may be further processed by adding a liquid, preferably distilled water to the powder to prepare a powder solution which is then stirred and homogenized by a homogenizer. The solution is centrifuged by a centrifugal separator to extract a supernatant which is then filtered for removal of suspended solid to finally obtain a medicine or chemicals of solution.

The seleniferous chelate can also be absorbed by garlic, broccoli or other agricultural products. When agricultural products are cultivated, seleniferous chelate fertilizer is applied in soil to harvest agricultural products grown with and including Se-methylselenocysteine or derivative thereof, γ-glutamylmethyl-selenocysteine and selenious acid.

Also, the seleniferous composition according to the present invention boosts to yield glutathione peroxidase (GPx) in lungs and glutathione-S-transferase (GST) in liver and kidney. Glutathione peroxidase (GPx) metabolizes hydrogen peroxide into water, and glutathione-S-transferase (GST) makes a catalysis of the chemical reaction between active oxygen and glutathione to detoxify carcinogen in the living body, and therefore, the composition according to the present invention can efficaciously prevent and treat liver cancer, hepatocirrhosis, kidney cancer, renal pelvic tumor and lung cancer.

The seleniferous composition produces in vivo a large amount of enzymes capable of resolving or separating carcinogen into harmless substances and thoroughly inhibits any generation, multiplication and metastasis of cancer cells to prevent and treat cancer. In addition, the seleniferous composition has so low toxicity as to be safe for living body so that a patient can take the composition in the increased amount for a long period of time while receiving the benefit of an excellent anticancer action without bad side effect.

### Example 1

A seleniferous compound was prepared which comprises by weight, 80 parts of Se-methylselenocysteine and 10 parts of glutamylmethylselenocysteine to measure the produced amount of superoxide dismutase (SOD), glutathione peroxidase (GPx) and glutathione-S-transferase (GST) enzymes and then finally determine the detoxifying effect by the enzymes. A similar measurement was also made to ascertain the amount of isozymes (Yb1, Yb2, Yp) which may catalyze a similar reaction as that catalyzed by glutathione-S-transferase, but has the different moleculars therefrom.

F344 female rats of 7-weeks old were fed for three weeks with two kinds of based diet with and without seleniferous composition, and then their liver, kidney and lungs were extracted. The obtained internal organs were mixed with 0.2 M sucrose-10 mM phosphate buffer solution of pH 7.4 in the amount three times of each weight of the organs, and consequently cut and broken into pieces by supersonic disintegrator to measure the amount of isozymes Yb1, Yb2, Yp of GST prepared in the internal organs. The mixed solution was centrifuged under 700G of centrifugal force to obtain a first supernatant which was then utilized for the activity measurements of SOD and GPx. The first supernatant was further centrifuged under 105,000G of centrifugal force to obtain a second supernatant for use in the activity measurement of GST.

The activity of SOD and GPx was measured with BIOXTECH GPX-340® Assay manufactured by Oxis International Inc. Also, the amount or activity of GST was calculated by measuring the change in light absorption of the wavelength 340 nm in the reaction solution at a temperature of 30 °C. The reaction solution contained the second supernatant, 1mM CDNB (1-chrolo-2,4-dinitrobenzene) as substrate, 100mM phosphate buffer (pH6.5), and reduced glutathione (GSH).

Then, GST isozymes Yb1, Yb2, Yp were measured by the Western Blot method as described below. Electrophoresis was performed to separate proteins from the mixed solution of the internal organ fragment utilizing 12.5 % SDS-polyacrylamide gel (SDS-PAGE). The separated protein was imprinted on a nitrocellulose membrane manufactured by Bio Rad, which was then subjected to blocking treatment with a 3% skimmed milk TBS buffer solution (50mM Tris-HCl, 200mM NaCl (pH7.4)), to react the imprinted protein with a rabbit anti-rat polyclonal antibody manufactured by Biotin International Limited, diluted 1/1000, for labeling GST M1 (Yb1), GST M2 (Yb2) and GST P1 (Yp), at a temperature of 4 °C overnight. Subsequently, the imprinted protein was sufficiently cleaned twice with TBS buffer solution and TBS buffer solution containing 0.05 % Tween 20, and was reacted with alkaline phosphatase-labeled goat anti-rabbit antibody IgG manufactured by Jacson Immuno Research Laboratories, Inc., diluted 1/5000 for an hour at room temperature. After the imprinted protein was further washed twice with TBS buffer solution and TBS buffer solution containing 0.05 % Tween 20, the protein was colored with a coloring reagent named "Nitro Blue Tetra" manufactured by Pierce Corporation to measure each intensity of the developed color bands of isozymes Yb1, Yb2, Yp with ATTO Lane & Spot Analyzer 5.0 manufactured by ATTO Corporation.

The result of these measurements indicated that the rats fed with diet and seleniferous composition generated larger amount of SOD and GST in the kidney, GST in the liver, and GPx in the lungs than that of rats fed with diet without the seleniferous composition. Also, inner organs of the former rats generated larger amount of isozymes Yb1 and Yb2 of glutathione-S-transferase in the liver than that of the latter rats. Thus, it has been found that the seleniferous composition according to the present invention promotes to develop antioxidases which perform the antioxidation action in kidney, liver and lungs so that the composition finally results in generating large amount of anti-oxidizing and anticancer agents.

### Example 2

Daikon sprouts were cultivated with seleniferous chelate fertilizer, and distilled water of 10ml was added to dried and powdered daikon sprouts of 1 gram by weight. The powder solution was stirred by a homogenizer and centrifuged to extract a supernatant which was then filtered by a filter with the 0.22 µm pore size for the separated identification of the seleniferous compound by means of an inductively coupled plasma mass spectrometry method of high performance liquid chromatograph (HPLC-ICPMS) utilizing Agilent 7500 manufactured by Yokogawa Analytical Systems and an anion exchange high performance liquid chromatograph column of Hamilton PRP X-100 manufactured by Hamilton Company. As a result of the above mass analysis, Figure 1 shows that the seleniferous compound of 100 % as a whole by weight basis leads to bear Se-methylselenocysteine of 82 %, γ-glutamylmethylselenocysteine of 10 %, and seleniferous acid of 8 % as shown by respectively peaks 1, 2 and 3.

F344 female rats of 7-weeks old were fed for three weeks with two kinds of daikon sprouts base diet with and without seleniferous composition, and then their liver, kidney and lungs were extracted, and then measured in a similar way as Example 1 for the produced amount of SOD, GPx and GST in their internal organs. Also, a test was carried out to measure the produced amount of isozymes Yb1, Yb2, Yp of GST.

Similarly to Example 1, rats fed with diet and seleniferous composition produced in vivo larger amounts of SOD and GST in the kidney, GST in the liver, and GPx in the lungs than that of rats fed with diet only. Also, larger amounts of isozymes Yb1 and Yb2 of GST in the liver were generated from their internal organs of rats with the seleniferous composition. In this way, it has been found that daikon sprouts including the seleniferous composition promotes to develop antioxidases as anti-oxidizing and carsinostatic agents in internal-organs.

The present invention should not be limited to the application to seleniferous composition for preventing or treating cancer, but can also be effectively applicable to prevent and treat any diseases, trouble, failure, deficiency, insufficiency, dysfunction and malfunction in vivo derived from active oxygen, for example, angiopathy such as arteriosclerosis, cerebral thrombosis, cerebral apoplexy, high blood pressure, disseminated intravascular coagulation (DIC), and leukemia; cardiopathy such as cardiomyopathy, myocardial infarction, and coronary heart disease; respiratory illuness such as adult respiratory distress syndrome (ARDS), and pulmonary emphysema; ophthalmophathy such as cataract, presbyopia, senile cataract, and premature infant retinitis; visceral disorder such as ulcerative colitis, hepatitis, acute pancreatitis, ischemic enteritis, nephritis, and drug hepatopathy; geriatrics such as wrinkle, senile pigment freckle, white hair, amnesia, dementia, and senility; and other disease such as stiff shoulder, Crohn disease, ultraviolet damage, autoimmune disease, shock, stress-caused ulcer, epileptic fit, frostbite, diabetes, Parkinson's disease, dropsy, radiation damage, and rheumatism.

## Claims

1. A seleniferous composition comprising a Se-methylselenocysteine or a derivative thereof.

2. A seleniferous composition according to claim 1, further comprising at least an amino compound.

3. A seleniferous composition according to claim 2, wherein said amino compound comprises one or more amino acid groups selected from the group consisting of glycine, alanine, valine, arginine, lysine, aspartic acid, glutaminic acid, serine, threonine, tyrosine, methionine and cystine.

4. A seleniferous composition according to- claim 2 or 3, said amino compound comprises at least a glutamylmethylselenocysteine.

5. A seleniferous composition according to claim 1 or 2, further comprising at least a selenious acid.

6. A seleniferous composition comprising, on the weight basis, 40 to 120 parts of a Se-methylselenocysteine or derivative thereof, and 4 to 20 parts of a glutamylmethylselenocysteine.

7. A seleniferous composition according to claim 6, further comprising 2 to 15 parts of at least a selenious acid.

8. A seleniferous composition according to claim 1 or 2, wherein said seleniferous composition is formed into chemicals shape of tablet, capsule, powder, granule or solution.

9. A seleniferous composition according to claim 1 or 2, wherein said composition serves to generate an enzyme including at least a superoxide dismutase when ingested in a living body.

10. A seleniferous composition according to claim 1 or 2, wherein said derivative of Se-methylselenocysteine has a metallic or nonmetallic element, or organic group.

11. A seleniferous composition according to claim 10, wherein said organic group is one or more selected from the group consisting of alkyl and aryl groups substituted for hydrogen of carboxyl group of Se-methylselenocysteine.

12. A seleniferous composition according to claim 1, further comprising γ-glutamylmethylselenocysteine.

13. A seleniferous composition according to claim 12, further comprising a selenious acid.

14. A seleniferous composition according to claim 13, wherein said sleniferous compound of 100 % as a whole by weight basis leads to bear Se-methylselenocysteine of about 82 %, γ-glutamylmethylselenocysteine of about 10 %, and seleniferous acid of about 8 %.

15. A method for preparing a seleniferous composition comprising the steps of:
cultivating plants with a seleniferous chelate fertilizer to grow agricultural products;
harvesting and drying said agricultural products; and
milling the dried agricultural products into powder.

16. A method for preparing a seleniferous composition according to claim 15, wherein said seleniferous chelate fertilizer includes a saccharic acid.

17. A method for preparing a seleniferous composition according to claim 15 or 16, further comprising forming said powder into a medicine or chemicals of tablet, capsule, powder, granule or solution for dissolution.

18. A method for preparing a seleniferous composition according to claim 15, further comprising:
adding a liquid to said powder to prepare a powder solution;
stirring and homogenizing said powder solution; and
centrifuging the homogenized powder solution to extract a supernatant thereof.

19. A method for preparing a seleniferous composition according to claim 18, wherein said liquid is distilled water.

20. A method for preparing- a seleniferous composition according to claim 18, further comprising filtering the extracted supernatant to obtain a medicine or chemicals of solution.

21. A method for preparing a seleniferous composition according to claim 15 or 18, wherein said plants is daikon sprouts, garlic or broccoli.

22. A method for preparing a seleniferous composition according to claim 15 or 18, wherein said agricultural product comprises a Se-methylselenocysteine or derivative thereof, a glutamylmethylselenocysteine and a selenious acid.
